# EUROPÄISCHE PATENTANMELDUNG

(11) **EP 2 374 820 A2**
(43) Veröffentlichungstag der Anmeldung: **12.10.2011**
(21) Anmeldenummer: 10194202.7
(22) Anmeldetag: 12.11.2004
(51) Int. Cl.: C07K 16/44, A61K 39/00

(54) **Humaner monoklonaler Antikörper mit fettsenkender Wirkung**

(30) Priorität: 14.11.2003 DE 10353175
(62) Teilanmeldung aus: 04802719.7
(71) Anmelder: Patrys Limited, Melbourne, Victoria 3000 (AU)
(72) Erfinder: Vollmers, Heinz Peter, 97078, Würzburg (DE)
(74) Vertreter: Cohausz & Florack

(57) **Zusammenfassung**

Aufgereinigtes Polypeptid, dessen Aminosäuresequenz im Wesentlichen identisch ist mit der Aminosäuresequenz von SEQ ID NO:1 und SEQ ID NO:3, wobei das Polypeptid low density lipoproteins (LDL) und/oder oxidiertes LDL (ox-LDL), insbesondere LDL-Cholesterin und/oder oxidiertes LDL-Cholesterin (oxLDL-Cholesterin), bindet. Die Erfindung beinhaltet die Verwendung des Polypeptides in Kombination mit üblichen Hilfs- und/oder Trägerstoffen zur Herstellung eines Arzneimittels mit fettsenkender Wirkung sowie von Medikamenten zur Behandlung von Nierenerkrankungen.

## Beschreibung

Die Erfindung betrifft ein aufgereinigtes Polypeptid (SAM-6.10) sowie dessen Verwendung in Kombination mit üblichen Hilfs- und/oder Trägerstoffen zur Herstellung eines Arzneimittels mit fettsenkender Wirkung sowie zur Herstellung eines Arzneimittels zur Behandlung von Nierenerkrankungen.

### Hintergrund der Erfindung

Bei einem an Überangebot an Cholesterin (Hyperlipoproteinämie) im Körper kommt es zur Verkalkung (arteriosklerotische Plaque) der Innenschichten der Gefäße und zu einer langsam fortschreitenden Verhärtung und Verdickung der Arterienwände. Im Extremfall droht ein Verschluss des Gefäßes oder beim Aufbrechen der Plaque Thrombusbildung. Die Arteriosklerose ist mit ihren Folgeerkrankungen (koronare Herzkrankheiten, Herzinfarkt, periphere arterielle Verschlusskrankheiten, Schlaganfall) noch immer die häufigste Todesursache in der westlichen Welt. Weit über die Hälfte aller für die medizinische Betreuung zur Verfügung stehenden fianziellen Mittel werden schätzungsweise für die Folgen der Arteriosklerose ausgegeben. Um die Ursachen der Arteriosklerose zu klären, wurden verschiedene Theorien entwickelt, wobei die Lipidtheorie die meistbeachtetste ist.

Allgemein läßt sich sagen: Je höher der LDL-Cholesteringehalt bzw. der Gehalt an oxidiertem LDL-Cholesterin im Blut, desto höher das Risiko, an einer Gefäßverkalkung beispielsweise mit der Folge eines Herzinfarktes zu erkranken. Übergewicht und Hypercholesterinämie sind mit die wichtigsten Risikofaktoren für die Entwicklung einer Arteriosklerose.

### Definitionen und Begriffe

Fette, wie z. B. Cholesterin, sind weder in Wasser noch in Blutflüssigkeit löslich. Um sie trotzdem in einzelne Körperregionen transportieren zu können, werden die Fette, sobald sie sich im Blut befinden, an bestimmte Eiweißkörper (Proteine) gebunden. Diese Verbindungen aus Lipiden (Fetten) und Proteinen (Eiweißen) werden als Lipoproteine bezeichnet.

Die "Lipoproteine" des Plasmas sind hochmolekulare wasserlösliche Komplexe, die aus Lipiden (Cholesterin, Triglyceride, Phospolipide) und Apolipoproteinen bestehen. Das cholesterinhaltige Lipoprotein LDL-Cholesterin verursacht Arteriosklerose und wird auch das "böse" Cholesterin genannt, wobei die oxidierte Form des LDL-Cholesterins für den Körper noch gefährlicher ist.

"Cholesterin" wird im Körper ubiquitär synthetisiert und ist ein wesentlicher Bestandteil von Zellmembranen und Lipoproteinen. lm Gegensatz zu den ebenfalls endogen synthetisierten Triglyceriden und Phospholipiden kann der Sterolring des Cholesterinmoleküls nicht mehr abgebaut werden; Cholesterin wird in der Leber zu Gallensäure umgewandelt oder unverändert über die Galle in den Darm ausgeschieden.

lm Plasma liegt Cholesterin zu 25-40% als freies (unverestertes) Cholesterin und zu 60-75% mit ungesättigten Fettsäuren verestert vor. Beide Formen zusammen werden als Gesamtchotesterin bezeichnet. Wegen seiner geringen Wasserlöslichkeit wird Cholesterin im Plasma als Komplex mit Apolipoproteinen transportiert. Im Blut werden etwa 70 Prozent des Gesamt-Cholesterins mit Hilfe von Low-Density-Lipoproteinen (LDL) transportiert.

"Triglyceride" sind Ester von Glycerin mit drei Fettsäureresten. Analog zum Cholesterin werden auch die Triglyceride im Plasma aufgrund ihrer schweren Löslichkeit an Apolipoproteine gebunden transportiert.

"Lipoproteine" werden in der Leber oder im Darm synthetisiert und transportieren im Blut fettlösliche Substanzen wie das Cholesterin.

Die Einteilung der Lipoproteine erfolgt nach ihrer Dichte, man unterscheidet die fünf Dichteklassen: Chylomikronen, very low density lipoproteines (VDL), low density lipoproteines (LDL) und high density lipoproteines (HDL). Die Chylomikronen, deren physiologische Konzentration im Nüchternserum im Gegensatz zu jenen der anderen Lipoproteine sehr gering ist, sind Transportvehikel für exogene Glyceride. Die physiologische Verteilung der anderen Lipoproteine ist wie folgt: VLDL 10 %, LDL 70 % und HDL 20 %. VLDL sind die Vorläufer der LDL und Vehikel für den Transport von endogenen Glyceriden. LDL entstehen durch die Hydrolyse der VLDL. LDL und HDL sind beides Regulatoren der zellulären Cholesterinhomöostase, wobei HDL außerdem die Lipolyse (Spaltung von Triglyzeriden in Glyzerin und die freien Fettsäuren) regulieren. LDL haben einen Durchmesser von ca. 20 nm. HDL sind die kleinsten (7-10 nm) und eiweißreichsten Lipoproteinen. Neben nativem LDL (LDL) ist im Blutserum auch oxidiertes LDL (oxLDL) nachweisbar. oxLDL interagiert mit endogenen Plasmaproteinen, insbesondere Glycoproteinen, durch spezifische Liganden und bildet oxLDL-Glykoprotein-Komplexe.

,,Apolipoproteine" sind ein Bestandteil der Lipoproteine und umgeben, zusammen mit polaren Lipiden, als eine Art äußere Schale den aus hydrophoben Lipiden aufgebauten Lipoprotein-Kern. Mit Ausnahme von LDL, welches nur Apoprotein B enthält, weisen die einzelnen Lipoproteinklassen mehrere strukturell verschiedene Apolipoproteinklassen auf.

### Lipoprotein-Transport

Cholesterin wird hauptsächlich durch die beiden Lipoproteinklassen LDL und HDL transportiert. LDL sind vor allem zuständig für den Chlosterintransport zu peripheren Zellen, die spezifische Rezeptoren für die LDL besitzen. Die HDL ermöglichen und beschleunigen den Abtransport von Cholesterin aus den extrahepatischen Zellen und Gefäßwänden und führen es der Leber zu.

### Pathogenität

Hinsichtlich der Pathogenität bei Lipidstoffwechselstörungen läßt sich allgemein sagen, dass LDL-Cholesterinerhöhungen in Verbindung mit HDL-Cholesterinverminderungen die ausgeprägteste Risikoerhöhung für Arteriosklerose darstellen. In der Pathogenese spielen LDL, deren Partikel wesentlich zur Bildung atherosklerotischer Plaques beitragen, und HDL daher eine gegensätzliche Rolle. Für die Beurteilung des Infarktrisikos sind die Quotienten aus Gesamtcholesterin/HDL-Cholesterin und insbesondere LDL-Cholesterin/HDL-Cholesterin entscheident. (Auf die schützende Wirkung des HDL-Cholesterins weisen auch epidemiologische Studien (Framingham-Studie) hin.) Die Folgeerkrankungen der Arteriosklerose beinhalten neben der koronaren Herzkrankheit und peripheren arteriellen Verschlußkrankheiten insbesondere Infarkte in Herz und Gehirn (Schlaganfall).

Vermutlich verursacht oxLDL genauso wie LDL arteriosklerotische Plaques, wobei für den Körper von oxLDL die größere Gefahr ausgeht.

Aber auch bei anderen Erkrankungen scheint oxLDL eine wichtige Rolle zu spielen. Bei Patienten mit chronischem Nierenversagen und Diabetes ist die Konzentration an oxLDL-Glykoprotein-Komplexen höher als bei gesunden Patienten.

LDL wird durch die Leber und durch Makrophagen aus dem Blutkreislauf entfernt. Makrophagen sind Zellen des Fremdkörperabwehrsystems, die zur Phagozytose größerer Partikel fähig sind.

Der "Scavenger-Pathway" ist ein bekanntes Modell zur Erklärung der Aufnahme von Partikeln durch Zellen (Phagozytose). Die Aufnahme fester Partikel (Gewebstrümmer, Fremdkörper, Bakterien oder LDL-Plaques) in das Zellinnere von Phagozyten mit nachfolgendem intrazellulären Abbau erfolgt durch Phagozytose. Die zu Phagozytose befähigten Zellen werden auch als Fresszellen bezeichnet und bestehen überwiegend aus Gewebsmakrophagen sowie aus mobilen Blutmonozyten.

Bei der "Phagozytose" kommt es nach Anlagerung der Partikel an die Zellmembranen der Phagozyten durch Bindung an membranständige Fc- und Komplementrezeptoren zur Aktivierung kontraktiler Strukturen innerhalb des Zytoplasmas. Durch lokale Einstülpungen der Zellmembranen kommt es zum Einschluss der Partikel in Zytoplasmavakuolen.

Die sog. Abräum-(Scavenger-)Phagozyten findet man im Lymphknoten in und entlang der zur Medulla (Mark) gehörenden Faserstränge. Während der Lymphpassage vom afferenten zum efferenten Ende des Lymphknotens werden partikuläre Antigene durch die zur Phagozytose befähigten Zellen entfernt.

Darüberhinaus ist bekannt, dass die Adhärenz an phagozytierende Zellen, wie polymorphkernige Leukozyten und Makrophagen, durch die Anhaftung von lmmunoglobulinen (Ig) an die Oberfläche von Bakterien (und anderen Antigenen) vergrößert ist. Es wird vermutet, dass die gesteigerte Adhärenz durch Anlagerung des Fc-Anteils des Immunoglobulins an die Fc-Rezeptoren der Phagozyten bewirkt wird. Nach dem "Schmackhaftmachen der Antigene durch Anhaftung von (od Bindung von) Antikörpern" wird der Komplex aus Antigen und Antikörpern von den phagozytierenden Zellen schneller aufgenommen und verdaut. Die Beschichtung der Antikörperoberfläche mit lmmunoglobulinen wird auch als opsonin-bedingte (Fc) Adhärenz bezeichnet und spielt eine wichtige Rolle bei der Immunabwehr.

Die an die Oberfläche von Bakterienzellen bindenden Antikörper sind in der Lage, bestimmte Komponenten der extrazellulären Flüssigkeiten zu fixieren. Im Überbegriff werden diese Komponenten als "Komplement" bezeichnet. Tierversuche zeigten, dass die Phagozytose von mit Antikörpern beschichteten Zellen bei jenen Tieren mit Komplementmangel verzögert ist. Somit liegt nahe, dass es bei der Opsonierung einen Synergismus zwischen Antikörpern und Komplement gibt.

### Beschreibung der Erfindung

Die Wirkung der aus dem Stand der Technik bekannten Arzneimittel zur Senkung des LDL-Cholesterins beruht auf der Hemmung des Schlüsselenzyms der Cholesterinsynthese (CSE). Als Cholesterinsynthesehemmer ist beispielsweise eine unter dem Handelsnamen Lipobay vermarktet Substanz bekannt geworden. Die Nebenwirkungen der CSE-Hemmer allgemein sind erheblich und schließen u.a. gastrointestinale Störungen, Schlafstörungen, Schwindel, Sehstörungen, allergische Reaktionen und Haarausfall ein. Lediglich im Versuchsstadium, und zwar nur bei schwerer familiärer Hypercholesterinämie, ist ein Ansatz der somatischen Gentherapie, der die Übertragung des Gens für den LDL-Rezeptor auf autologe Leberzellen zum Inhalt hat.

Ein weitgehend nebenwirkungsfreier Stoff, der als Fettsenker wirkt, ist bislang nicht auf dem Markt. Insbesondere sind Antikörper, die eine verstärkte intrazelluläre Akkumulation von Lipoproteinen induzieren, bislang nicht bekannt. Obwohl die schädigende Rolle von Lipoproteinen bei Nierenerkrankungen (Lipid-induzierte Schädigung von Zellen des Glomerulus-Filtrations-Apparates der Niere) bekannt ist, gibt es noch keinen auf Antikörper basierenden Therapie-Ansatz von Nieren Erkrankungen, insbesondere der Glomerulonekrose,

Die Aufgabe der Erfindung besteht in der Generierung eines neuen Stoffes bzw. einer neuen Stoffklasse zur Herstellung eines Arzneimittels zur Verringerung des LDL-Cholesterins bzw. des oxLDL-Cholesterins bei Mensch und Tier mit dem vorteilhaften Ziel der Verringerung des Infarktrisikos.

Zur Lösung der Aufgabe wird ein Polypeptid vorgeschlagen, dessen
- dessen Aminosäuresequenz im wesentlichen identisch ist mit der Aminosäuresequenz von SEQ ID NO:1 und/oder SEQ ID NO:3, und
- das low density lipoproteins (LDL) und/oder oxidiertes LDL (oxLDL), insbesondere LDL-Cholesterin und/oder oxidiertes LDL-Cholesterin (oxLDL-Cholesterin), bindet.

Der Kern der Erfindung besteht in der überraschend gemachten Beobachtung, dass ein gereinigtes Polypeptid, dessen Sequenz ganz oder teilweise der leichten (V_{L}) oder schweren Kette (V_{H}) eines humanen monoklonalen Antikörpers (SAM-6.10) entspricht, die Senkung des low density lipoproteins (LDL) und/oder oxLDLs bewirkt. Die Entdeckung dieser Eigenschaft, die den Einsatz des Polypeptids in entsprechender pharmazeutischer Formulierung als Fettsenker nahelegt, wurde im Rahmen der biochemischen Charakterisierung des Polypeptids gemacht. Vorteilhafterweise ist die Bindung des erfindungsgemäßen Polypeptids oder der Fragmente des Polypeptids an LDL und/oder oxLDL und an VLDL, den Vorläufern der LDL, stärker ist als die Bindung an HDL. Aufgrund dieser Eigenschaft führt das erfindungsgemäße Polypeptid zu einem geringen Wert für den jeweiligen Quotienten aus LDL/HDL und/oder oxLDL/HDL und minimiert somit das Infarktrisiko.

Die spezifische Bindung des Polypeptides an low density lipoproteins (LDL und/oder oxLDL), bzw. LDL-Cholesterin und/oder oxLDL-Cholesterin wurde experimentell durch die ELISA-Methode nachgewiesen. Im gleichen Experiment konnte auch gezeigt werden, dass die Bindung des erfindungsgemäßen Stoffes an high density lipoproteins (HDL) schwach ist.

Der erfindungsgemäße Antikörper umfasst die nach der gängigen Nomenklatur zur Beschreibung von Antikörper bezeichneten Gruppen V_{L} ,V_{H}, Fv, Fc, Fab, Fab', F(ab')₂. Die genannten Gruppen werden auch als Fragmente bezeichnet. Es ist durchaus möglich, dass ein einzelnes Fragment Ursache für die fettsenkende Wirkung des erfindungsgemäßen Polypeptids ist. In einer Weiterbildung des erfindungsgemäßen Stoffes handelt es sich um einen human monoklonalen Antikörper.

Als "funktionelles Fragment" im Sinne der Erfindung wird ein Polypeptid bezeichnet, dass zumindest eine der biologischen Aktivitäten besitzt, die auch das gesamte Polypeptid aufweist. Bei Antikörpern ist z. B. bekannt, dass für die spezifische Bindung nicht alle CDR-Regionen erforderlich sind. D.h. die spezifische Bindung des Antikörpers kann z. B. durch nur eine CD-Region bewerkstelligt werden, obwohl insgesamt 3 CD-Regionen vorhanden sind. Die spezifische Bindung des Antikörpers an ein Antigen kann z.B. zur Induktion von Apoptose oder zur Inhibition der Zellprolieferation führen. Die biologische Aktivität eines funktionellen Fragmentes kann durch verschiedene, dem Fachmann bekannte Methoden, gemessen werden. Eine Methode zur Messung der Interaktion zwischen Antikörpern und LDL, insbesondere LDL-Cholesterin, ist die ELISA-Methode.

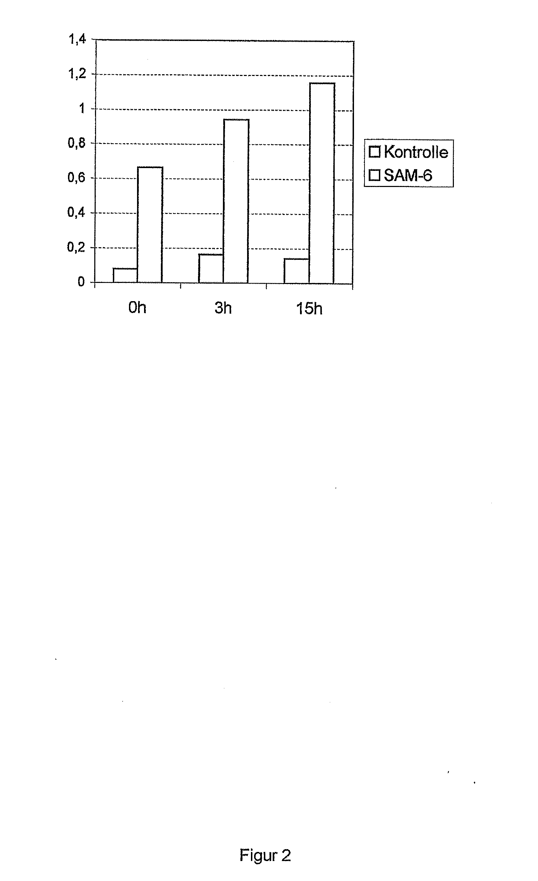

Die complementartity-determining regions (CDRs) der Polypeptidsequenz beinhaltet die Aminosäuresequenz, die im wesentlichen identisch ist mit der Aminosäurensequenz Ser-Gly-Asp-Lys-Leu-Gly-Asp-Lys-Tyr-Ala-Cys (CDR1), Gln-Asp-Ser-Lys-Arg-Pro-Ser (CDR2) und Gin-Ala-TrpAsp-Ser-Ser-lle-Val-Val (CDR3) der SEQ ID NO 1 der variablen Region der leichten Kette (\/_{L}); siehe auch Figur 2.

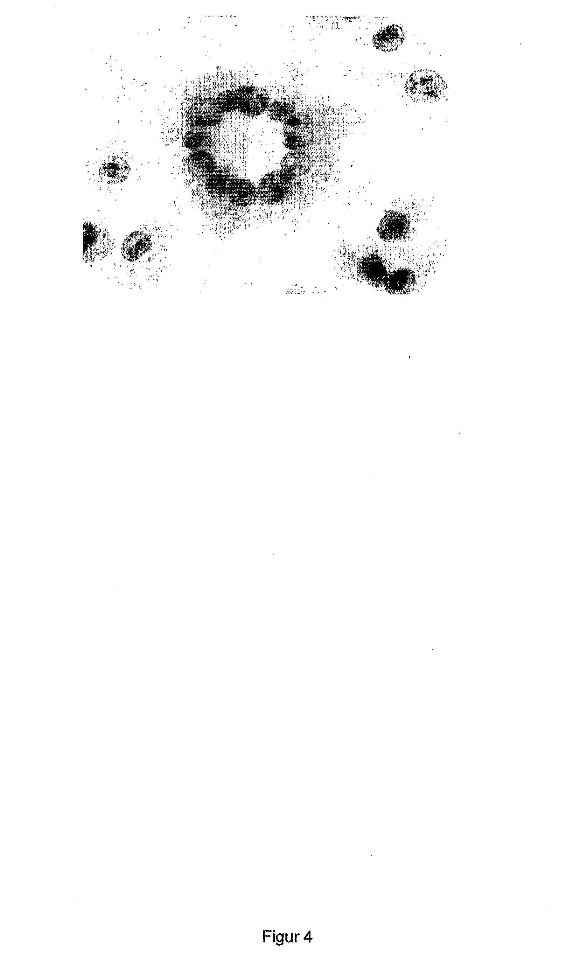

Die complementarity-determining regions (CDRs) der Peptidsequenz beinhalten Aminosäuresequenzen, die im wesentlichen identisch sind mit Ser-Tyr-Ala-Met-His (CDR1), Val-lle-Ser-Tyr-Asp-Gly-Ser-Asn-Lys-Tyr-Tyr-Ala-Asp₋Ser-Val-Lys-Gly (CDR2) und Asp-Arg-Leu-Ala-Val-Ala-Gly-Lys-Thr-Phe-Asp-Tyr (CDR3) der SEQ ID NO 3 der variablen Region leichten Kette (V_{H}); siehe auch Figur 4.

Als "im wesentlichen identisch" wird ein Polypeptid oder eine Nukleinsäuresequenz bezeichnet, die zumindest 75 %, 80 %, 85 %, oder 90 % mit der als Referenz angegebenen Aminosäurensequenz (SEQ ID IO: 1 und 3) oder mit der Nukleinsäurensequenz (SEQ ID NO: 2 und 4) aufweist. In einer Weiterbildung des Polypeptids bzw. der Nukleinsäurensequenz sind mindestens 95 %, 98 %, 99 % oder 100 % Identität im Vergleich zu den angegebenen Referenzen nachweisbar. Für Polypeptide wird die Länge des Vergleichsabschnitts im allgemeinen mindestens 5, 10, 15 oder wünschenswerterweise mindestens 20 oder 25 aufeinander folgende Aminosäuren aufweisen.

Das erfindungsgemäße Polypeptid ist generierbar durch ein Verfahren, das unter dem Namen Hypridoma-Technik (Köhler, Millstein, Nature, 1975, Vol. 256, 495) bekannt ist und die Isolation von monoklonalen Antikörpern ermöglicht. Es beruht auf der in vitro-Gewinnung von zellulären Hybriden die durch Zellfusion von normalen Lymphozyten mit unbegrenzt lebens- und teilungsfähigen Myelomzellen (z.B. HAB-1) gewonnen werden. Die hierbei erzeugten Hybridom-Zellen weisen Eigenschaften beider Elternzellen auf. Dem entsprechend besitzen sie die Fähigkeit der Lymphozyten, Antikörper zu produzieren (z.B. SAM-6.10) und die Fähigkeit der Myelomzelle zur unbegrenzten Teilung und damit zur Produktion der Antikörper in großen Mengen. Jede aus der Fusion resultierende Hybridzelle stellt monoklonale Antikörper her deren Spezifität von der ursprünglichen Lymphozytenzelle bestimmt wird. Die Hybridomzellen werden vermehrt und dann diejenigen selektiert, welche Antikörper der gewünschten Spezifität produzieren. Die Kultivierung dieser Auswahl und deren Isolierung führt zu hochspezifisch reagierenden Antikörpern, welche nur mit einer bestimmten antigenen Determinante reagieren.

Der Nachweis der deutlichen Senkung des low density lipoprotein (LDL)-Spiegels (bzw. des LDL-Cholesterin Spiegels) bzw. der jeweils oxidierten Form im Blutplasma wurde im Tierexperiment bestätigt, ohne dass sich der HDL-Spiegel im nachweisbaren Bereich senkt. Wesentlich dabei ist, dass die Vitalfunktionen der Tiere während der Gabe des Antikörpers nicht beeinträchtigt werden, so dass die erfindungsgemäße Substanz bislang als Nebenwirkungsfrei bezeichnet werden kann. (Die Wirkungsweise des erfindungsgemäßen Antikörpers könnte sich in Analogie zum Mechanismus des bekannten Scavenger-Pathways erklären lassen.)

Eine weiteres Indikationsgebiet für den Einsatz des erfindungsgemäßen Medikamentes ist die Behandlung von Nierenerkrankungen, insbesondere die Glomerulonekrose (Glomerulosklerose).

Es liegt im Rahmen der Erfindung, dass das erfindungsgemäße Polypeptid zur Herstellung des Arzneimittels bevorzugt in gereinigter Form eingesetzt wird, wobei zur Reinigung sämtliche dem Fachmann bekannten Verfahren (z.B. Affinitätschromatographie, Gelfiltration) in Frage kommen. Als Indikation des erfindungsgemäßen Stoffes steht des fettsenkende Wirkung im Vordergrund, wobei insbesondere die selektive Senkung von LDL bzw. LDL-Cholesterin hervorzuheben ist. Aufgrund der Eigenschaft LDL und/oder oxLDL stärker zu binden als HDL bewirkt das erfindungsgemäße Polypeptid einen geringen Wert für den Quotienten aus LDL/HDL und/oder oxLDL/HDL und minimiert somit das Infarktrisiko.

Die Hilfs- und Trägerstoffe zur Herstellung eines Arzneimittels sind dem Fachmann bekannt und können nach gängiger Praxis hergestellt werden (siehe Remington: The Science and Practice of Pharmacy (20th ed.), ed. A.R. Gennaro, Lippincott Williams & Wilkins, 2000 and Encyclopedia of Pharmaceutical Technology, eds. J. Swarbrick and J. C. Boylan, 1988-1999, Marcel Dekker, New York).

### Material und Methoden

### Immortalisierung von Lymphozyten und Primärtestung der Antikörper

Zur Immortalisierung werden die Lymphozyten mit dem Heteromyelom HAB-1 (Faller et al., 1990) nach Standardprotokoll fusioniert und kultiviert. Kurz zusammengefasst, Lymphozyten werden mit HAB-1 Zellen mittels PEG verschmolzen. Die Triome werden auf vier 24-Lochplatten ausgesät. Die durchschnittliche Wachstumsfrequenz beträgt 80-90%, 50% der wachsenden Klone sezernieren Immunglobuline. Die erste Austestung der sezernierten humanen monoklonalen Antikörper erfolgt im ELISA, um den Isotyp zu ermitteln. Nachfolgend können die humanen monoklonalen Antikörper immunhistochemisch, genetisch, biochemisch und molekularbiologisch Analysiert werden.

Benötigte Medien:
- RPMI 1640 (Firma PAA) ohne Zusätze RPMI 1640 mit HAT-Zusatz (HAT-Supplement, Firma PAA) sowie 10% FCS, 1 % Glutamin und 1 % Penicillin/Streptomycin
- HAB-1 (Fusionspartner) zweimal mit RPMI ohne Zusätze waschen
- zentrifugieren 5 min bei 1500U/min
- eingefrorene Lymphozyten (aus Milz, Lymphknoten oder Blut) auftauen und zweimal mit RPMI ohne Zusätze waschen, ebenfalls zentrifugieren
- beide Pellets jeweils in 10 ml RPMI ohne Zusatz aufnehmen und in der Neubauer-Zählkammer zählen
- im Verhältnis von 1:2 - 1:3, Hab-1 zu Lymphozyten, fusionieren
- die Zellpellets nach dem zweiten Waschvorgang zusammen geben, mischen und 8 min bei 1500 U/Min zentrifugieren
- das zuvor bei 37°C aufgewärmte PEG (Polyethylene Glycol 1500, Firma Roche) vorsichtig tröpfelweise auf das Pellet unter leicht rotierenden Bewegungen des 50 ml Röhrchens laufen lassen
- leicht resuspendieren und dann genau 90 sek. im Wasserbad bei 37°C rotieren lassen
- danach wir das PEG mit RPMI ohne Zusätze herausrausgewaschen (zwei volle 10er Pipetten)
- zentrifugieren 5 min bei 1500 U/min
- 24 Well-Platten ausplattieren mit 1 ml pro Well RPMI mit HAT-Zusatz (HAT= Hypoxanthin, Aminopterin, Thymidin)
- das Pellet lösen in RPMI mit HAT-Zusatz
- jeweils einen halben ml der Zellen in ein 24 Well pipettieren
- Fusionsplatten in den Brutschrank stellen
- wöchentlich Mediumwechsel mit RPMI mit HAT-Zusatz

### Reinigung des Antikörpers SAM-6.10

Reinigung von Kulturüberstand durch Kationenaustauschchromatographie über FPLC

Die den IgM-Antikörper SAM-6.10 produzierenden Hybridomzellen wurden hierzu in einem speziellen serumfreien Zellkulturmedium (AIMV-Medium, Gibco) herangezogen und der Gehalt an IgM im Kulturüberstand nephelometrisch bestimmt. Zur Aufreinigung wurde der Kulturüberstand auf einen pH-Wert von auf 5,9 eingestellt und die Lösung filtriert. Zur Bindung wurde eine spezielle Kationen-Säule (HiTrap™ SP FF column, 5 ml, Amersham Bioscience) verwendet. Die Säule wurde zu Beginn der Reinigung mit filtriertem Puffer A (20 mM Phosphatpuffer, pH 5,9) äquilibriert. Anschließend wurde der auf Eis gekühlte Kulturüberstand mit einer Flussrate von 1 ml/min auf die Säule aufgetragen. Nach dem Auftrag des Überstandes wurde die Säule für 20 min und einer Flussrate von 2 ml/min mit Puffer A bis zu einer Basislinienkonstanz gewaschen um alle nicht gebundenen Proteine zu entfernen. Anschließend wurde der an die Säule gebundenen Antikörper durch Beimischung von Puffer B (20 mM Phosphatpuffer, 1 M NaCI, pH 8,0) eluiert und in Fraktionen gesammelt. Der Gehalt an SAM-6.10 Antikörper (IgM) in den einzelnen Fraktionen wurde im Folgenden nephelometrisch bestimmt und die Reinheit und Intaktheit des gereinigten Antikörpers über SDS-PAGE und Western-Blot Analyse überprüft.

### Messung von oxLDL

Die Messung erfolgt mit Hilfe des Test-Kits Oxidised LDL ELISA von der Firma Mercodia, Uppsala, Schweden. Test-Prinzip: Der ELISA für oxidiertes LDL ist ein enzymatischer, zweiseitiger Immunoassay mit einer Festphase. Er basiert auf der direkten Sandwich-Technik, bei der zwei monoklonale Antikörper gegen ein bestimmtes Antigen auf oxidiertem ApolipoproteinB ausgerichtet werden. Während der Inkubation reagiert oxidiertes LDL in der Probe mit anti-oxidiertem LDL-Antikörpern, die an die Vertiefungen in der Mikrotiterplatte gebunden sind. Nach dem Waschen, wobei die nicht-reaktiven Bestandteile der Probe entfernt werden, erkennt ein peroxidasekonjugierter anti-human Apolipoprotein B Antikörper das an der festen Phase gebundene oxidierte LDL. Nach einer zweiten Inkubation und einem Waschvorgang, der die mit dem ungebundenen Enzym markierten Antikörper entfernt, wird das Konjugat durch Reaktion mit 3, 3', 5, 5'-Tetramethylbenzidin (TMB) nachgewiesen. Die Reaktion wird durch Zugabe von Säure gestoppt, um einen kolorimetrischen Endpunkt zu definieren der bei 450 nm spektrophotometrisch abgelesen wird.

### Messung der Bindung von SAM-6 an oxLDL

Eine ELISA-Platte (Becton Dickinson Labware Europ, Frankreich) wurde mit den unterschiedlich stark oxidierten LDL Fraktionen über Nacht bei 4°C inkubiert. Die unspezifischen Bindungsstellen wurden mit RPMI-1640 Medium welches mit 10% FCS versetzt wurde für 1 h geblockt. Anschließend wurde die Platte mit 60µg/ml SAM-6 Antikörper für eine Stunde bei 37°C inkubiert. Nach dreimaligem Waschen mit PBS wurde die ELISA-Platte für 1 h mit HRP-gekoppeltem Zweitantikörper (rabbit anti human IgM, Dako, Hamburg, Deutschland, 1:1000 in PBS) inkubiert. Danach wurde die Platte nochmals mit PBS und Citratpuffer gewaschen, mit OPD (DakoCytomation, Glostrup, Dänemark) versetzt und der Farbumschlag bei 490 nm mit einem ELlSA-reader gemessen.

### Durchflusszytometrie (FACS Analyse)

Die verwendeten adhärenten Zellen wurden durch Behandlung mit Trypsin/EDTA vom Boden der Kulturflaschen abgelöst. Die Reaktion wurde mit 10 ml RPMI-1640 Medium (+ Supplemente) abgestoppt und die Zellen mit 1000 x g für 5 min pelletiert. Die Zellen wurden zweimal mit PBS gewaschen, in FACS-Puffer (PBS, 0,01% Na-Azid) aufgenommen und zur Rekonstitution der Zellmembranen für 30 min auf Eis gestellt. Anschließend wurden die Zellen auf eine Dichte von 1 x 10⁶ Zellen/ml eingestellt und je 200 µl der Zellsuspensionen in FACS-Reaktionsgefäße überführt, sodass pro Röhrchen eine Zellzahl von 2 x 10⁵ Zellen vorlag. Die Zellen wurden für 5 min bei 4°C und 1400 rpm pelletiert, die Pellets resuspendiert und mit den Primär-Antikörpern für 15 min auf Eis inkubiert. Als Primär-Antikörper wurden 100 µg/ml SAM-6 Antikörper in FACS-Puffer (Gesamtvolumen 200 µl) bzw. 100mg/ml LDL verwendet. Als Isotyp-Kontrolle fungierten 100 µg/ml Chrompure human IgM. Alternativ wurden die Zellen mit LDL für 30 min vorinkubiert und anschließend für 15 min 100µg/ml SAM-6 Antikörper zugesetzt.

Nach der Inkubation wurden die Zellen abzentrifugiert, der Überstand verworfen und die Pellets mit 500 µl kaltem FACS-Puffer gewaschen. Danach erfolgte eine 15minütige lichtgeschützte Inkubation mit dem FITC-gekoppelten Sekundär-Antikörper (Rabbit anti human IgM, FITC-gekoppelt, 1:50 in FACS-Puffer für SAM-6 bzw. Chrompure human lgM). Nach einem erneuten Waschgang wurden die Zellen in 200 µl kaltem FACS-Puffer aufgenommen und bis zur Messung lichtgeschützt auf Eis aufbewahrt. Die Messung erfolgte mittels eines Durchflußzytometers (FACScan; Becton Dickinson, USA).

### Tierexperimente zum Nachweis der in-vivo Wirkung des Antikörpers SAM-6.10

**Experiment 1:** 500µg gereinigter SAM-6.1 0 Antikörper wurden intra peritoneal injiziert. Die LDL Konzentration im Blutserum wurde nach 2 Tagen gemessen (Methode siehe unten).

Experiment 2: wie oben

**Experiment 3:** 1 mg gereinigter SAM-6.10 Antikörper wurden intrapereitoneal injiziert. Die LDL Konzentration im Blutserum wurde nach 14 Tagen gemessen.
Kontrolle A: Normalwerte Kontrollmaus
Kontrolle B: Normalwerte Kontrollmaus
Die Serumkonzentration von LDL ist bei den mit SAM-6.1 0 behandelten Mäusen deutlich reduziert (siehe Figur 10 und 11).

### Toxizität

500µg bzw. 1 mg gereinigter SAM-6.10 Antikörper wurde Mäusen intraperitoneal injiziert.
- keine akute Toxizität
- keine latente Toxizität (Zeitraum 3 Monate).

Die Organe der getöteten Mäuse aus Experiment 1, 2 und 3 (siehe oben) wurden entnommen und untersucht: Leber, Lunge, Herz, Milz, Dünndarm, Dickdarm, Nieren, Magen und Gehirn wiesen keine morphologischen Veränderungen auf. Darüber hinaus wurden die Organe immunhistochemisch auf eventuelle Lipideinlagerungen untersucht. Die Färbung mit Sudan III zeigte in keinem Organ eine Einlagerung von Lipiden.

Die Organe der getöteten Mäuse wurden in Formalin fixiert und in Paraffin eingebettet. Die Färbung erfolgte mit dem Farbstoff Sudan III nach folgendem Protokoll:

### Sudan III-Färbung auf Parafffinschnitten/ auf Makrophagen

Entparaffinierung:
- Xylol 1 5 min
- Xylol 2 5 min
- 100% Ethanol 1 5 min
- 100% Ethanol 2 5 min
- Methanol 70ml
   + H₂O₂ 500µl 5 min
- 90% Ethanol 1 3 min
- 90% Ethanol 2 3 min
- 80% Ethanol 1 3 min
- 80% Ethanol 2 3min
- 70% Ethanol 1 3 min
- 70% Ethanol 2 3 min
- Schnitte in PBS stellen
- Schnitte 15 min mit Sudan III inkubieren
- waschen mit Aqua dest.
- 1 x in 60% Isopropanol eintauchen
- waschen mit Aqua dest.
- Gegenfärbung mit Hämalaun für 6 min
- Schnitte 10 min wässern, waschen mit Aqua dest und mit Glyceringelatine eindeckeln

Für die Sudan III Färbung von Makrophagen werden die adherenten Makrophagen auf Objekträgern angezüchtet und dann mit den entsprechenden Agenzien behandelt. Die Färbung wird wie folgt durchgeführt:
- Fixierung der Zellen in 60% Isopropanol (6 min)
- 20 min mit Sudan III inkubieren
- waschen mit Aqua dest.
- Gegenfärbung mit Hämalaun für 6 min
- Schnitte 10 min wässern, waschen mit Aqua dest und mit Glyceringelatine eindeckeln

### Bestimmung von Lipiden in Blutproben

Die Messung der verschiedenen Lipide im Blutserum wurde automatisch mit dem MODULAR D P800 Gerät (Roche) vorgenommen.

Die Bestimmung des LDL-Cholesterinwertes erfolgte durch eine enzymatische, kolorimetrische Methode (CHOD/PAP) ohne Probenvorbehandlung.

**Testprinzip zur Bestimmung der Lipidkonzentration im Serum:** HD L, VLDL und Chylomikronen werden von einem Detergenz 1 spezifisch hydrolysiert. Das in diesen Lipoproteinen freigesetzte Cholesterin reagiert sofort durch die enzymatische Wirkung der Cholesterinesterase (CE) und Cholesterinoxidase (CHOD) und es entsteht Wasserstoffperoxid. Dies bildet mit 4-Aminoantipyridin in Anwesenheit einer Peroxidase (POD) ein farbloses Produkt. Während dieses Schrittes bleiben die LDL-Partikel intakt. Die Reaktion von LDL-Cholesterin wird durch Zugabe von Detergenz 2 und der Kupplungssubstanz N,N-bis(4-Sulfobutyl)-m-toluidin (DSBmT) ausgelöst. Das zweite etergenz setzt Cholesterin in den LDL-Partikeln frei. In der enzymatischen Reaktion wird in Anwesenheit der Kupplungssubstanz ein Farbstoff gebildet. Die Intensität des gebildeten roten Chinoniminfarbstoffs ist direkt proportional zur LDL-Cholesterin-Konzentration. Sie wird durch Messung der Extinktionszunahme bei 552 nm bestimmt.

### ELISA (LDL/HDL)

- ELISA-Platte vorbeschichten mit LDL (Lipoprotein Low Densi tyfrom Human Plasma, Firma Sigma, 10µg/ml in PBS) oder HDL (Human HDL, Firma Chemicon ,10µg/ml in PBS) → 50 µl pro well
- Platte abdecken und über Nacht bei 4°C lagern
- am nächsten Tag Platte 2 x mit PBS waschen
- 100 µl RPMI in jedes well pipettieren. und für 1h bei RT inkubieren
- danach 2 x mit PBS waschen
- 50 µl der jeweiligen Positivkontrollen in je 2 wells pipettieren (Doppelbestimmung)
   Positivkontrolle: Mab to human LDL Mouse lgG2a 1:1000 in PBS
- daneben 50 µl RPMI als Negativkontrolle (Doppelbestimmung)
- 50 µl der Proben (Überstand SAM6.10) (Doppelbestimmung) nebeneinander pipettieren
- 1 h im Brutschrank inkubieren
- 2 x mit PBS waschen
- 2 x mit PBS/0,05% Tween waschen
- 2 x mit PBS waschen
- 50 µl der jeweiligen 2. AK (Peroxidase konjugiert.) pipettieren:
   rabbit anti human lgM 1:1000 in PBS/0,05%Tween (für SAM-6.10)
   rabbit anti Mouse lgGs 1:1000 mit PBS Tween (für Positivkontrolle LDL)
- 1 h im Brutschrank inkubieren
- 2 x mit PBS waschen
- 1 x mit PBS/0,05% Tween waschen
- 2 x mit PBS waschen
- 2 x mit Citratpuffer waschen
- zum Auswerten: OPD Tablette (Dako, Hamburg) in Citratpuffer lösen + H₂O₂ (3 ml Citratpuffer + eine Tabl. + 5 µl H₂O₂)
- 50 µl Farbstoff in jedes Well pipettieren
- bei positiver Reaktion (gelbe Färbung) mit 10 µl 3 M H₂SO₄ stoppen

### Erläuterung der Figuren

### Sequence listing

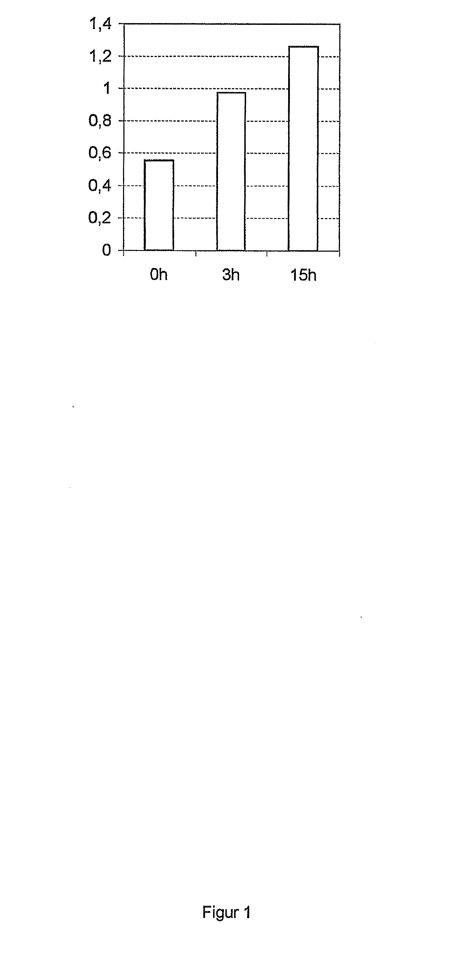

Figur 1 zeigt die Aminosäuresequenzen (SEQ ID NO 1) der variablen Region der leichten Kette (V_{L}).
Figur 2 zeigt die Nucleotidsäuresequenz (SEQ ID NO 2) der variablen Region der leichten Kette (V_{L}). Die complementarity-determining regions (CDR) sind durch Querstriche gekennzeichnet und im wesentlichen identisch mit den Nucleotiden 67-99 (CDR1), 145-165 (CDR2) und 262-288 (CDR3) von SEQ ID NO 2.

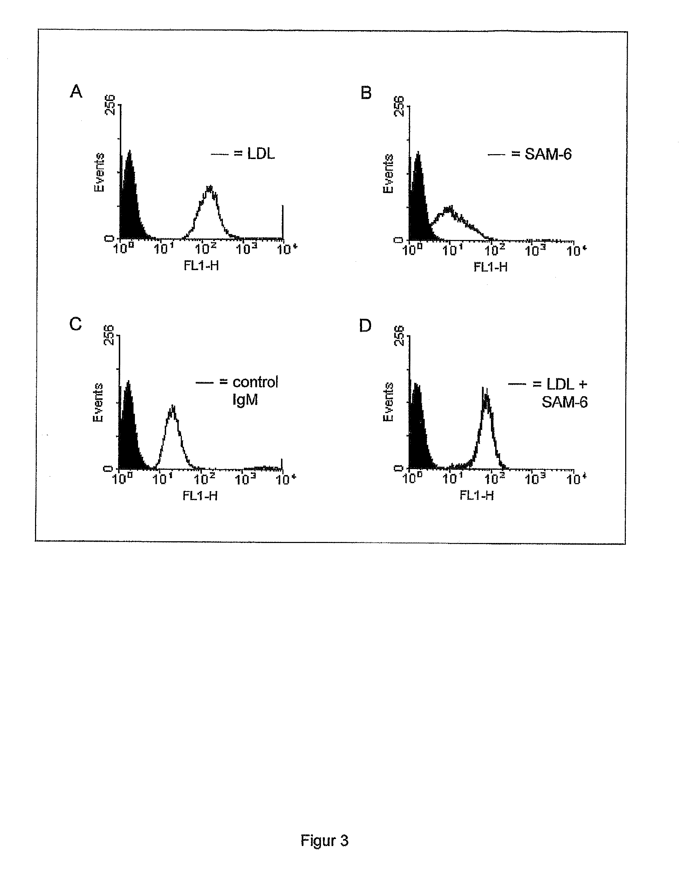

Figur 3 zeigt die Aminosäuresequenzen (SEQ ID NO 3) der variablen Region der schweren Kette (V_{H}).
Figur 4 zeigt die Nucleotidsäuresequenz (SEQ ID NO 4) der variablen Region der schweren Kette (V_{H}). Die complementarity-determining regions (CDR) sind durch Querstriche gekennzeichnet und im wesentlichen identisch mit den Nucleotiden 91-105 (CDR1), 148-198 (CDR2) und 295-330 (CDR3) von SEQ ID NO 4.

### Zellbiologische Experimente

Die nachfolgende erklärten Figuren sollen die Erfindung nicht einschränken, sondern nur erläutern und deren Ausführbarkeit beispielhaft belegen.

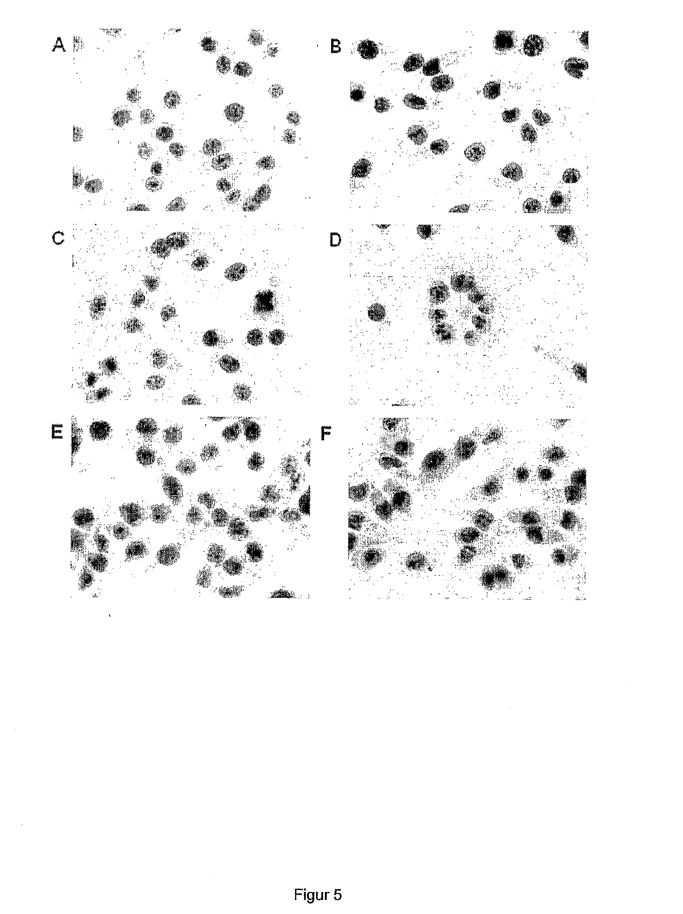

Figur 5 zeigt die Messung von oxLDL in Abhängigkeit von der Inkubationdauer mit einer Kupfersulfatlösung. Im Versuch wurde LDL (Sigma, Taufkirchen, Deutschland) wurde für 3 bzw. 15h mittels Inkubation mit 20µM CuSO₄ oxidiert. Mit dem Mercodia Oxidised LDL ELISA wurde, der Gebrauchsanweisung folgend, die Menge an oxidiertem LDL bestimmt. Deutlich zu erkennen ist, dass die Menge an oxidiertem LDL mit steigender Inkubationsdauer zunimmt, wobei auch jene LDL Fraktion, welche nicht mit Kupferionen behandelt wurde, zum Teil schon in oxidierter Form vorliegt. Nach 15 stündiger Inkubation ist der Anteil an oxidiertem LDL etwa verdoppelt.

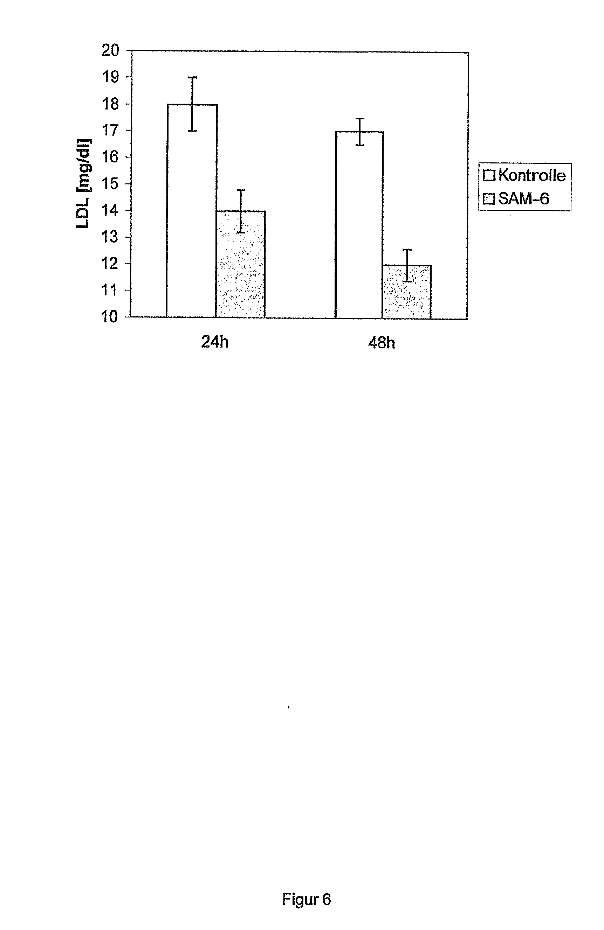

Figur 6 zeigt den Nachweis der Bindung von SAM-6 an oxLDL. Für den Nachweis der Bindung von SAM-6 an oxLDL mittels ELISA Bindungsassay wurden die ELISA Platte mit unterschiedlich stark oxidierten LDL Fraktionen vorbeschichtet, bevor der Erstantikörper SAM-6 und der zu Detektionszwecken erforderliche Zweitantikörper anti-human IgM hinzugegeben wurde. Das Ergebnis zeigt, dass je mehr LDL in seiner oxidierten Form vorliegt, desto stärker bindet der erfindungsgemäße Antikörper SAM-6.

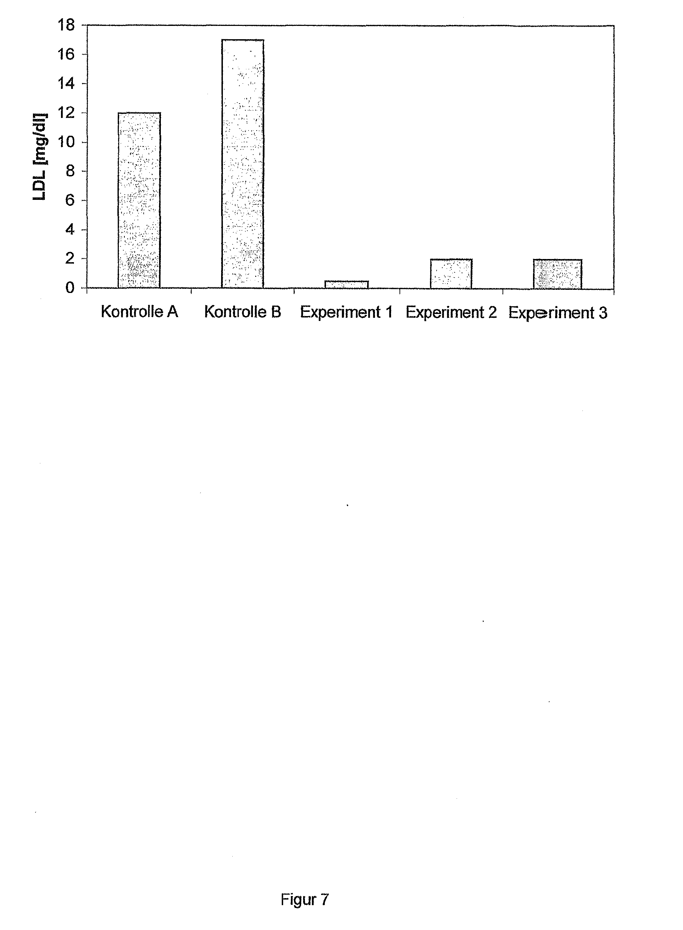

Figur 7 zeigt das Ergebnis einer FACS Analyse. Bei den dafür verwendeten Zellen handelt es sich um Zellen der Maus-Makrophagen Zellinie P388D1(IL-1) (DSMZ Accession No. ACC 288). Figur 7A zeigt die Bindung von LDL an Macrophagen. Figur 7B belegt, dass auch der humane monoklonale Antikörper SAM-6 an Macrophagen bindet. Mit dem Nachweis der Bindung eines Kontroll IgM an Macrophagen in Figur 7C wird gezeigt, dass die Macrophagen µ-Rezeptoren besitzen. Mit der Rechtsverschiebung des Signals in Figur 7D wird gezeigt, dass eine gleichzeitige Inkubation von LDL und SAM-6 eine vermehrte Bindung von SAM-6 an die Zellen bewirkt.

Die Figuren 8 und 9 zeigt das Ergebnis der Sudan III Färbung für die Zellen der Maus-Makrophagen Zellinie P388D1(IL-1) verwendet wurden.

In Figur 8 sind Zellen gezeigt, die für 48h entweder mit SAM-6 oder einem IgM Kontrollantikörper inkubiert und anschließend einer Sudan III Färbung unterzogen wurden. Die mit dem Antikörper SAM-6 inkubierten Zellen zeigen aufgrund der Rotfärbung eine deutliche Einlagerung von Neutralfetten. Die mit dem Kontroll-Antikörper inkubierten Zellen zeigen dagegen keine Veränderungen.

Für die in Figur 9 zeigten Färbungen wurden die Makrophagen für 24h sowohl mit als auch ohne Zusatz von FCS kultiviert. Danach wurde für weitere 24h entweder nur LDL oder nur SAM-6 beziehungsweise LDL und SAM-6 gemeinsam zugesetzt. Anschließend wurde eine Sudan III Färbung durchgeführt. Die linke Bildspalte mit den Figuren 9A, 9C und 9E zeigen Zellen, die ohne den Zusatz von FCS kultiviert wurden. Die rechte Bildspalte mit den Figuren 9B, 9D und 9F zeigen Zellen, die mit Zusatz von FCS kultiviert wurden.

Die Figuren 9A und 9B belegen, dass sowohl Makrophagen, die ohne FCS kultiviert wurden, als auch Macrophagen, die in Anwesentheit von FCS gewachsen sind, eine basale Einlagerung von Neutralfetten zeigen. Die Figur 9C belegt, dass bei Zugabe von SAM-6 zu Makrophagen, die ohne FCS kultiviert wurden, keine Fett-Einlagerung nachweisbar ist. Dagegen wird, wie in Figur 9D gezeigt, eine verstärkte Lipid-Akkumulation bei Makrophagen beobachtet, die mit FCS kultiviert wurden und denen anschliessend SAM-6 zugesetzt wurde. Die Figuren 9E und 9F zeigen, das bei einer Co-Inkubation von SAM-6 und LDL die intrazelluläre Lipid-Akkumulation sowohl bei Makrophagen, die mit FCS kultiviert wurden als auch bei denen die ohne Zusatz von FCS gewachsen sind, deutlich zu nimmt.

Figur 10 zeigt den Einfluss des Antikörpers SAM-6 auf LDL Werte *in vivo.* Im Versuch wurde den Mäusen 1mg gereinigter SAM-6 Antikörper bzw. im Kontrollexperiement 1 mg human Chrompure IgM (Isotyp-Kontrolle) i.p. injiziert. Die LDL Konzentration im Blut wurde nach 24 und 48h gemessen. Dabei kann nach 24 und 48h Stunden bei SAM-6 behandelten Mäusen eine deutliche Reduktion von Serum LDL beobachtet werden.

Die Messung von LDL im Blutserum wurde automatisch mit dem MODULAR D P800 Gerät (Roche) vorgenommen, wobei sich die aufgetragenen Werte als Ergebnis des Diagnostik Kits "LDL-Cholesterol Direct" (Firma Roche Diagnostiks) ergeben.

Figur 11 weist ebenfalls die *in vivo* Wirkung von SAM-6.10 nach, wobei zur Errechnung der Werte eine indirekte Methode nach der Friedewald-Formel angewandt wurde. Dabei errechnet sich die Menge an LDL aus der Differenz von Gesamtcholesterin, HDL und Triglyceriden. Gemäß dieser Art der Versuchsauswertung wäre die Reduktion der Konzentration von LDL im Serum nach SAM-6.10 Behandlung noch stärker.. Diese indirekte Meßmethode muss jedoch im Vergleich zu der in Figur 10 angewandten Methode als ungenauer angesehen werden.

### Ausführungsformen

1. Gereinigtes Polypeptid, **dadurch gekennzeichnet,** dass
   - die Aminosäuresequenz des Polypeptids im wesentlichen identisch ist mit der Aminosäuresequenz von SEQ ID NO:1 und/oder SEQ ID NO:3, und
   - das Polypeptid low density lipoproteins (LDL) und/oder oxidiertes LDL (oxLDL), insbesondere LDL-Cholesterin und/oder oxidiertes LDL-Cholesterin (oxLDL-Cholesterin), bindet.
2. Gereinigtes Polypeptid nach Ausführungsform 1, **dadurch gekennzeichnet,** dass die Bindung des Polypeptides oder der Fragmente des Polypeptids an low density lipoproteins (LDL) und very low density lipoproteins (VLDL), bzw. die jeweils oxidierte Form (oxLDL und (oxVLDL), stärker ist als die Bindung an high density lipoproteins (HDL).
3. Polypeptid nach einer der vorhergehenden Ausführungsformen **dadurch gekennzeichnet,** dass das Polypeptid oder Fragmente des Polypeptids und die im menschlichen und tierischen Körper vorkommenden low density lipoproteins (LDL) und/oder oxidierten low density lipoproteins (oxLDL) komplementäre Carbohydrat-Strukturen aufweisen.
4. Gereinigtes Polypeptid nach einer der vorhergehenden Ausführungsformen
   **dadurch gekennzeichnet,** dass das Polypeptid ein Antikörper oder ein funktionelles Fragment davon ist.
5. Gereinigtes Polypeptid nach einer der vorhergehenden Ausführungsformen
   **dadurch gekennzeichnet,** dass das Polypeptid ein funktionelles Fragment einer der nachfolgend genannten Gruppen von V_{L}, V_{H}, Fv, F_{c}, Fab, Fab', und F(ab')₂ ist.
6. Gereinigtes Polypeptid nach Ausführungsformen 5, **dadurch gekennzeichnet,** dass die Aminosäuresequenz der variablen Region der leichten Kette (V_{L}) im wesentlichen identisch ist mit SEQ ID NO 1 und/oder die Aminosäuresequenz der variablen Region der schweren Kette (V_{H}) im wesentlichen identisch ist mit SEQ ID NO 3.
7. Gereinigtes Polypeptid nach Ausführungsform 5, **dadurch gekennzeichnet,** dass die Nucleinsäuresequenz der variablen Region der leichten Kette (V_{L}) im wesentlichen identisch ist mit SEQ ID NO 2 und/oder die Nucleinsäuresequenz der variablen Region der schweren Kette (V_{H}) im wesentlichen identisch ist mit SEQ ID NO 4.
8. Gereinigtes Polypeptid nach Ausführungsform 5, **dadurch gekennzeichnet,** dass die genannten Fragmente ein Fragment der Aminosäuresequenz der SEQ ID NO 1 oder der SEQ ID NO 3 beinhalten.
9. Gereinigtes Polypeptid nach Ausführungsform 5, **dadurch gekennzeichnet,** dass die genannten Fragmente ein Sequenzfragment enthalten, das im wesentlichen identisch ist mit der Aminosäuresequenz von SEQ ID NO 1 oder SEQ ID NO 3.
10. Gereinigtes Polypeptid nach Ausführungsform 1, **dadurch gekennzeichnet,** dass das Polypeptid im wesentlichen identisch ist mit der Aminosäuresequenz von SEQ ID NO 1.
11. Gereinigtes Polypeptid nach Ausführungsform 1, **dadurch gekennzeichnet,** dass das Polypeptid im wesentlichen identisch ist mit der Aminosäuresequenz von SEQ ID NO 3.
12. Gereinigtes Polypeptid nach einer der Ausführungsformen 1 und 3, **dadurch gekennzeichnet,** dass das Polypeptid Nucleinsäuresequenzen beinhaltet, die im wesentlichen identisch sind mit den Nucleotiden 67-99 (CDR1), 145-165 (CDR2) und 262-288 (CDR3) of SEQ ID NO 2.
13. Gereinigtes Polypeptid nach einer der Ausführungsformen 1 und 3, **dadurch gekennzeichnet,** dass das Polypeptid Nucleinsäuresequenzen beinhaltet, die im wesentlichen identisch sind mit den Nucleotiden 91-105 (CDR1), 148-198 (CDR2) and 295-330 (CDR3) of SEQ ID NO 4.
14. Gereinigtes Polypeptid, das die Aminosäuresequenz von SEQ ID NO:1 beinhaltet.
15. Gereinigtes Polypeptid, das die Aminosäuresequenz von SEQ ID NO:3 beinhaltet.
16.Gereinigtes Polypeptid, das die Aminosäuresequenz von SEQ ID NO:1 und/oder SEQ ID NO:3 beinhaltet.
17. Complementarity-determining regions (CDR) oder funktionelle Fragmente davon, welche die Aminosäuresequenzen Ser-Gly-Asp-Lys-Leu-Gly-Asp-Lys-Tyr-Ala-Cys (CDR1) oder Gln-Asp-Ser-Lys-Arg-Pro-Ser (CDR2) oder Gln-Ala-Trp-Asp-Ser-Ser-lle-Val-Val (CDR3) of SEQ ID NO: 1 und/oder Ser-Tyr-Ala-Met-His (CDR1) oder Val-lle-Ser-Tyr-Asp-Gly-Ser-Asn-Lys-Tyr-Tyr-Ala-Asp-Ser-Val-Lys-Gly (CDR2) oder Asp-Arg-Leu-Ala-Val-Ala-Gly-Lys-Thr-Phe-Asp-Tyr (CDR3) SEQ ID NO: 3 umfassen.
18. Gereinigtes Polypeptid nach einer der vorhergehenden Ausführungsformen **dadurch gekennzeichnet,** dass das Polypeptid nach einer der vorhergeheden Ausführungsformen 19 beanspruchten Verfahren generierbar ist. der in Ausführungsform
19. Verfahren zur Generierung eine Antikörpers nach der Hybridomtechnik, **dadurch gekennzeichnet,** dass die Hybridom-Zellen durch Fusion der Heteromyelom-Zellen HAB-1 sowie deren Subklone mit B-Lymphozyten gewonnen werden, welche aus humanen Milzen, Lymphknoten oder Blut entnommen sind.
20. Gereinigtes Polypeptid nach einer der Ausführungsformen 1, 17 und 19, **dadurch gekennzeichnet,** dass das Polypeptid ein monoklonaler Antikörper ist.
21. Gereinigtes Polypeptid nach Ausführungsform 20, **dadurch gekennzeichnet,** dass das Polypeptid ein humaner monoklonaler Antikörper ist.
22. Verwendung eines Polypeptids nach einer der vorhergeheden Ausführungsformen in Kombination mit üblichen Hilfs- und/oder Trägerstoffen zur Herstellung eines Arzneimittels mit fettsenkender Wirkung.
23. Verwendung des Polypeptids nach einer der Ausführungsformen 1 bis 22 zur Herstellung eines Arzneimittels zur Senkung von low density lipoproteins (LDL) und/oder oxidiertem LDL (oxLDL) im Blut.
24.Verwendung des Polypeptids nach einer der Ausführungsformen 1 bis 22 zur Herstellung eines Arzneimittels zur Senkung von LDL-Cholesterin und/oder oxidiertem LDL-Cholesterin (oxLDL-Cholesterin).
25. Verwendung des Polypeptids nach einer der Ausführungsformen 1 bis 22 zur Herstellung eines Arzneimittels zur Behandlung von Nierenerkrankungen.
26. Verwendung des Polypeptids nach Ausführungsform 25 zur Herstellung eines Arzneimittels zur Behandlung der Glomerulonekrose.

Sequence listing

<110> Vollmers, Philip

<120> Human monoclonal antibody

<150> DE-10 353 175.0
<151> 2003-11-14

<160> 4

<210> 1
<211> 96
<212> DNA
<213> Homo sapiens

<220>
<223> Amino acid sequence of the variable region of the light chain (VL) of antibody SAM-6

<400> 1

Ser Tyr Val Leu Thr Gln Pro Pro Ser Val Ser Val Ser Pro Gly
1               5                   10                  15

Gln Thr Ala Ser Ile Thr Cys Ser Gly Asp Lys Leu Gly Asp Lys
                20                  25                  30

Tyr Ala Cys Trp Tyr Gln Gln Lys Pro Gly Gln Ser Pro Val Leu
                35                  40                  45

Val Ile Tyr Gln Asp Ser Lys Arg Pro Ser Gly Ile Pro Glu Arg
                50                  55                  60

Phe Ser Gly Ser Asn Ser Gly Asn Thr Ala Thr Leu Thr Ile Ser
                65                  70                  75

Gly Thr Gln Ala Met Asp Glu Ala Asp Tyr Tyr Cys Gln Ala Trp
                80                  85                  90

Asp Ser Ser Ile Val Val
                95

<210> 2
<211> 288
<212> DNA
<213> Homo sapiens

<220>
<223> Nucleotide sequence of the variable region of the light chain (VL) of antibody SAM-6

<400> 2

tcc tat gtg ctg act cag cca ccc tca gtg tcc gtg tcc cca gga          45
Ser Tyr Val Leu Thr Gln Pro Pro Ser Val Ser Val Ser Pro Gly
 1               5                  10                  15

                                          CDR1
cag aca gcc agc atc acc tgc tct gga gat aaa ttg ggg gat aaa          90
Gln Thr Ala Ser Ile Thr Cys Ser Gly Asp Lys Leu Gly Asp Lys
                20                  25                  30

tat gct tgc tgg tat cag cag aag cca ggc cag tcc cct gtg ctg          135
Tyr Ala Cys Trp Tyr Gln Gln Lys Pro Gly Gln Ser Pro Val Leu
                35                  40                  45

                      CDR2
gtc atc tat caa gat agc aag cgg ccc tca ggg atc cct gag cga          180
Val Ile Tyr Gln Asp Ser Lys Arg Pro Ser Gly Ile Pro Glu Arg
                50                  55                  60

ttc tct ggc tcc aac tct ggg aac aca gcc act ctg acc atc agc          225
Phe Ser Gly Ser Asn Ser Gly Asn Thr Ala Thr Leu Thr Ile Ser
                65                  70                  75

ggg acc cag gct atg gat gag gct gac tat tac tgt cag gcg tgg          270
Gly Thr Gln Ala Met Asp Glu Ala Asp Tyr Tyr Cys Gln Ala Trp
                80                  85                  90

        CDR3
gac agc agc att gtg gta                                              288
Asp Ser Ser Ile Val Val
                95

<210> 3
<211> 110
<212> DNA
<213> Homo sapiens

<220>
<223> Amino acid sequence of the variable region of the heavy chain (VH) of antibody SAM-6

<400> 3

Gln Val Gln Leu Val Glu Ser Gly Gly Gly Val Val Gln Pro Gly
1               5                   10                  15

Arg Ser Leu Arg Leu Ser Cys Ala Ala Ser Gly Phe Thr Phe Ser
                20                  25                  30

Ser Tyr Ala Met His Trp Val Arg Glu Ala Pro Gly Lys Gly Leu
                35                  40                  45

Glu Trp Val Ala Val Ile Ser Tyr Asp Gly Ser Asn Lys Tyr Tyr
                50                  55                  60

Ala Asp Ser Val Lys Gly Arg Phe Thr Ile Ser Arg Asp Asn Ser
                65                  70                  75

Lys Asn Thr Leu Tyr Leu Gln Met Asn Ser Leu Arg Ala Glu Asp
                80                  85                  90

Thr Ala Val Tyr Tyr Cys Ala Arg Asp Arg Leu Ala Val Ala Gly
                95                  100                 105

Lys Thr Phe Asp Tyr
                110

<210> 4
<211> 330
<212> DNA
<213> Homo sapiens

<220>
<223> Nucleotide sequence of the variable region of the heavy chain (VH) of antibody SAM-6

<400> 4

cag gtg cag ctg gtg gag tct ggg gga ggc gtg gtc cag cct ggg     45
Gln Val Gln Leu Val Glu Ser Gly Gly Gly Val Val Gln Pro Gly
1               5                   10                  15

agg tcc ctg aga ctc tcc tgt gca gcc tct gga ttc acc ttc agt     90
Arg Ser Leu Arg Leu Ser Cys Ala Ala Ser Gly Phe Thr Phe Ser
                20                  25                  30

CDR1
agc tat gct atg cac tgg gtc cgc cag gct cca ggc aag ggg ctg     135
Ser Tyr Ala Met His Trp Val Arg Glu Ala Pro Gly Lys Gly Leu
                35                  40                  45

CDR2
gag tgg gtg gca gtt ata tca tat gat gga agc aat aaa tac tac     180
Glu Trp Val Ala Val Ile Ser Tyr Asp Gly Ser Asn Lys Tyr Tyr
                50                  55                  60

gca gac tcc gtg aag ggc cga ttc acc atc tcc aga gac aat tcc     225
Ala Asp Ser Val Lys Gly Arg Phe Thr Ile Ser Arg Asp Asn Ser
                65                  70                  75

aag aac acg ctg tat ctg caa atg aac agc ctg aga gct gag gac     270
Lys Asn Thr Leu Tyr Leu Gln Met Asn Ser Leu Arg Ala Glu Asp
                80                  85                  90

CDR3
acg gct gtg tat tac tgt gcg aga gat cgg tta gca gtg gct ggt     315
Thr Ala Val Tyr Tyr Cys Ala Arg Asp Arg Leu Ala Val Ala Gly
                95                  100                 105

aaa act ttt gac tac
Lys Thr Phe Asp Tyr
                110

## Patentansprüche

1. Gereinigtes Polypeptid, **dadurch gekennzeichnet, dass** es eine Aminosäuresequenz umfasst, die zu wenigstens 75 % identisch mit der Aminosäuresequenz SEQ ID:3 ist und wobei das Polypeptid an Low Density Lipoprotein (LDL), oxidiertes LDL (oxLDL), LDL-Cholesterol oder oxidiertes LDL-Cholesterol bindet.

2. Gereinigtes Polypeptid nach Anspruch 1, **dadurch gekennzeichnet, dass** die Bindung des Polypeptids an Low Density Lipoprotein (LDL), oxidiertes LDL (oxLDL), LDL-Cholesterol oder oxidiertes LDL-Cholesterol stärker ist als die Bindung an High Density Lipoproteine.

3. Das gereinigte Polypeptid nach Anspruch 1 oder 2, **dadurch gekennzeichnet, dass** die Low Density Lipoproteine (LDL) oder die oxidierten LDL (oxLDL) im menschlichen oder tierischen Körper voderkommen und komplementäre Kohlenhydrat-Strukturen aufweisen.

4. Ein gereinigter Antikörper oder ein funktionelles Fragment davon, **dadurch gekennzeichnet, dass** er das Polypeptid nach einem der Ansprüche 1 bis 3 umfasst.

5. Der gereinigte Antikörper oder dessen funktionelles Fragment nach Anspruch 4, worin das funktionelle Fragment ausgewählt ist aus der Gruppe, bestehend aus V_{H}, Fᵥ, Fab, Fab' und F (ab') ₂ •

6. Der gereinigte Antikörper oder dessen funktionelles Fragment nach Anspruch 4, **dadurch gekennzeichnet, dass** der Antikörper oder dessen funktionelles Fragment eine schwere Kette (V_{H}) enthält, die zu wenigstens 75 % identisch mit SEQ ID NO:3 ist.

7. Der gereinigte Antikörper oder dessen funktionelles Fragment nach Anspruch 4, **dadurch gekennzeichnet, dass** der Antikörper oder dessen funktionelles Fragment eine leichte Kette (V_{L}) enthält, die zu wenigstens 75 % identisch zu SEQ ID NO:1 ist.

8. Ein gereinigter Antikörper oder dessen funktionelles Fragment, **dadurch gekennzeichnet, dass** die Sequenz der variablen Region der leichten Kette (V_{L}) oder die Sequenz der variablen Region der schweren Kette (V_{H}) eine Complementary-Determining Region (CDR) umfasst, die ausgewählt ist aus Ser-Gly-Asp-Lys-Leu-Gly-Asp-Lys-Tyr-Ala-Cys (CDR1), den Aminosäuren 23 bis 33 der SEQ ID NO:1, oder Gln-Asp-Ser-Lys-Arg-Pro-Ser (CDR2) den Aminosäuren 49 bis 55 der SEQ ID NO:1, oder Gln-Ala-TrpAsp-Ser-Ser-Ile-Val-Val (CDR3) den Aminosäuren 88 bis 96 der SEQ ID NO:1, oder Ser-Tyr-Ala-Met-His (CDR1) den Aminosäuren 31 bis 35 der SEQ ID NO:3, oder Val-Ile-Ser-Tyr-Asp-Gly-Ser-Asn-Lys-Tyr-Tyr-Ala-Asp-Ser-Val-Lys-Gly (CDR2) den Aminosäuren 50 bis 66 der SEQ ID NO:3, oder Asp-Arg-Leu-Ala-Val-Ala-Gly-Arg-Pro-Phe-Asp-Tyr (CDR3) den Aminosäuren 99 bis 110 der SEQ ID NO:3.

9. Der gereinigte Antikörper oder dessen funktionelles Fragment nach Anspruch 8, **dadurch gekennzeichnet, dass** die Sequenz der variablen Region der leichten Kette (V_{L}) oder die Sequenz der variablen Region der schweren Kette (V_{H}) Ser-Gly-Asp-Lys-Leu-Gly-Asp-Lys-Tyr-Ala-Cys (CDR1), den Aminosäuren 23 bis 33 der SEQ ID NO:1, Gln-Asp-Ser-Lys-Arg-Pro-Ser (CDR2), den Aminosäuren 49 bis 55 der SEQ ID NO:1 und Gln-Ala-Trp-Asp-Ser-Ser-Ile-Val-Val (CDR3), den Aminosäuren 88 bis 96 der SEQ ID NO:1, oder Ser-Tyr-Ala-Met-His (CDR1), den Aminosäuren 31 bis 35 der SEQ ID NO:3, Val-Ile-Ser-Tyr-Asp-Gly-Ser-Asn-Lys-Tyr-Tyr-Ala-Asp-Ser-Val-Lys-Gly (CDR2), den Aminosäuren 50 bis 66 der SEQ ID NO:3, and Asp-Arg-Leu-Ala-Val-Ala-Gly-Arg-Pro-Phe-Asp-Tyr (CDR3), den Aminosäuren 99 bis 110 der SEQ ID NO:3 umfasst.

10. Ein gereinigter Antikörper oder dessen funktionelles Fragment, **dadurch gekennzeichnet, dass** die Sequenz der variablen Region der schweren Kette (V_{H}) Ser-Tyr-Ala-Met-His (CDR1), den Aminosäuren 31 bis 35 der SEQ ID NO:3, Val-Ile-Ser-Tyr-Asp-Gly-Ser-Asn-Lys-Tyr-Tyr-Ala-Asp-Ser-Val-Lys-Gly (CDR2), den Aminosäuren 50 bis 66 der SEQ ID NO:3, and Asp-Arg-Leu-Ala-Val-Ala-Gly-_Arg-Pro-Phe-Asp-Tyr (CDR3), den Aminosäuren 99 bis 110 der SEQ ID NO:3 umfasst.

11. Der gereinigte Antikörper oder dessen funktionelles Fragment nach einem der Ansprüche 4 bis 10, **dadurch gekennzeichnet, dass** der Antikörper oder dessen funktionelles Fragment an Low Density Lipoprotein (LDL), oxidiertes LDL (oxLDL), LDL-Cholesterol oder oxidiertes LDL-Cholesterol bindet.

12. Gereinigter Antikörper oder dessen funktionelles Fragment nach einem der Ansprüche 4 bis 10, **dadurch gekennzeichnet, dass** der Antikörper oder dessen funktionelles Fragment ein monoklonaler Antikörper ist.

13. Der gereinigte Antikörper oder dessen funktionelles Fragment nach einem der Ansprüche 4 bis 10, **dadurch gekennzeichnet, dass** der Antikörper oder dessen funktionelles Fragment ein menschlicher monoklonaler Antikörper ist.

14. Das Polypeptid nach einem der Ansprüche 1 bis 3 oder der Antikörper oder dessen funktionelles Fragment nach einem der Ansprüche 4 bis 10 zur Verwendung in der Verringerung von LDL oder LDL-Cholesterol.

15. Polypeptid nach einem der Ansprüche 1 bis 3 oder ein Antikörper oder dessen funktionales Fragment nach einem der Ansprüche 4 bis 10 zur Behandlung von Nierenerkrankungen.
